(19) **Europäisches Patentamt**
European Patent Office
Office européen des brevets

(11) **EP 1 554 569 B1**

(12) **EUROPÄISCHE PATENTSCHRIFT**

(45) Veröffentlichungstag und Bekanntmachung des Hinweises auf die Patenterteilung:
**11.06.2008 Patentblatt 2008/24**

(21) Anmeldenummer: **03772214.7**

(22) Anmeldetag: **14.10.2003**

(51) Int Cl.:
**G01N 27/414** (2006.01)    **G01N 33/487** (2006.01)

(86) Internationale Anmeldenummer:
**PCT/EP2003/011381**

(87) Internationale Veröffentlichungsnummer:
**WO 2004/036203 (29.04.2004 Gazette 2004/18)**

(54) **SENSOR-ANORDNUNG UND VERFAHREN ZUM BETREIBEN EINER SENSOR-ANORDNUNG**

SENSOR ARRANGEMENT AND METHOD FOR OPERATING A SENSOR ARRANGEMENT

SYSTEME DETECTEUR ET MODE DE FONCTIONNEMENT DE CE DISPOSITIF DETECTEUR

(84) Benannte Vertragsstaaten:
**FR GB**

(30) Priorität: **14.10.2002 DE 10247889**

(43) Veröffentlichungstag der Anmeldung:
**20.07.2005 Patentblatt 2005/29**

(73) Patentinhaber: **Infineon Technologies AG**
**81669 München (DE)**

(72) Erfinder:
• **EVERSMANN, Björn-Oliver**
**80336 München (DE)**
• **PAULUS, Christian**
**82362 Weilheim (DE)**

• **THEWES, Roland**
**82194 Gröbenzell (DE)**

(74) Vertreter: **Dokter, Eric-Michael**
**Viering, Jentschura & Partner**
**Patent- und Rechtsanwälte**
**Postfach 22 14 43**
**80504 München (DE)**

(56) Entgegenhaltungen:
**EP-A- 0 241 991        WO-A-88/08972**
**DE-A- 10 151 020        FR-A- 2 779 826**
**US-A- 4 322 680        US-A- 4 514 263**
**US-A1- 2001 044 177**

EP 1 554 569 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung betrifft eine Sensor-Anordnung und ein Verfahren zum Betreiben einer Sensor-Anordnung.

Die Schnittstelle zwischen der Biologie und der Halbleitertechnik ist aus wissenschaftlicher und wirtschaftlicher Sicht interessant. In diesem Zusammenhang ist beispielsweise die Kopplung zwischen biologischen Zellverbänden, wie beispielsweise Neuronen, und der Silizium-Mikroelektronik zu nennen. Ein biologisches System kann auf der Oberfläche eines halbleitertechnologischen Sensors mit matrixförmig angeordneten Sensor-Elementen orts- bzw. zeitaufgelöst untersucht werden. Insbesondere können Stoffwechselparameter der Zellen beispielsweise mittels Erfassens lokaler pH-Werte mithilfe von ionensensitiven Feldeffekttransistoren (ISFETs) als Sensor-Elementen aufgenommen werden. Ein ISFET weist eine ionensensitive Schicht in Wirkkontakt mit zu erfassenden elektrisch geladenen Partikeln auf, wobei die elektrisch geladenen Partikel die Leitfähigkeit des ISFETs charakteristisch beeinflussen, was als Sensorgröße erfassbar ist.

Von großem Interesse ist das Untersuchen der Reaktion eines biologischen Systems auf eine elektrische Stimulation. Neuronen (Nervenzellen) können über Ionenkanäle in den Zellmembranen in bestimmten Bereichen ihrer Oberfläche einen geringen elektrischen Strom erzeugen, der von einem darunter angeordneten Sensor detektiert wird. Die hierfür erforderlichen hohen Anforderungen an Orts- und Zeitauflösung des Sensors werden mittels der Silizium-Mikroelektronik erreicht.

Auch in anderen wichtigen Gebieten wie der medizinischen Analytik oder der DNA-Sensorik werden matrixförmige Anordnungen von Sensor-Elementen verwendet.

Für diese und andere mögliche Anwendungen von Halbleiter-Sensoren in integrierten Schaltkreisen ist es vorteilhaft, eine große Anzahl von Sensoren in einem gemeinsamen Sensor-Array zu integrieren. Bereits bei kleinen (und umso mehr bei großen) Sensor-Arrays kann nicht jeder Sensor an eine eigene Auslese-Schaltung angeschlossen werden. Die Anzahl von hierfür erforderlichen Leitungen sowie die Redundanz der außerhalb des Arrays angeordneten Auslese-Schaltungen sprechen gegen eine solche Lösung.

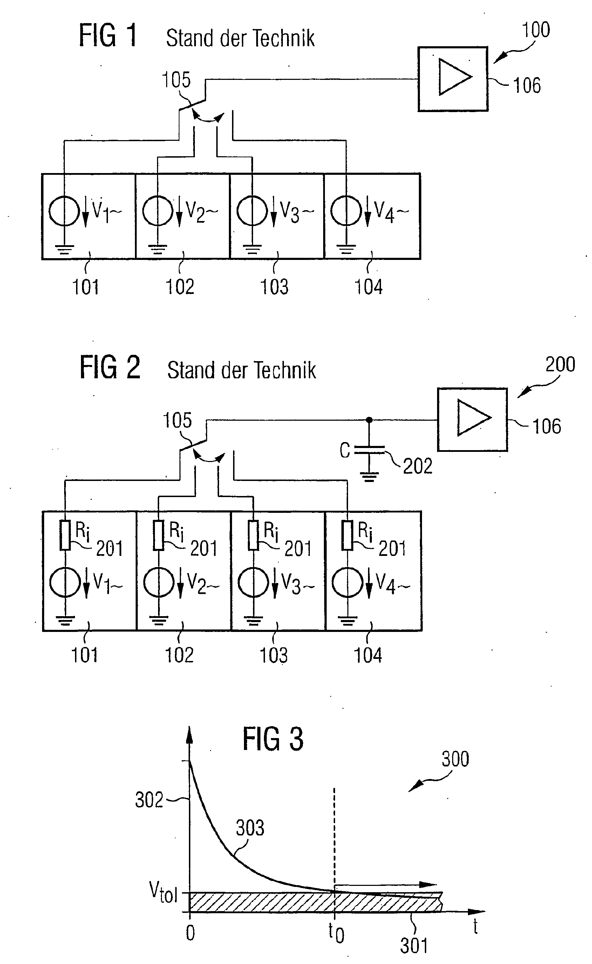

In **Fig.1** ist schematisch eine Sensor-Anordnung 100 gemäß dem Stand der Technik mit vier Sensor-Einrichtungen 101 bis 104, einem als Schalter 105 dargestellten Multiplexer und einer Auslese-Schaltung 106 gezeigt.

Die Ausgänge der einzelnen Sensor-Einrichtungen werden unter Verwendung des Multiplexers 105, das heißt seriell, ausgelesen. Die jeweilige über den Multiplexer 105 mit der Auslese-Schaltung 106 gekoppelte Sensor-Einrichtung 101 bis 104 gibt eine mit dem zu detektierenden Ereignis (beispielsweise die Beleuchtungsintensität bei einem Photosensor oder ein elektrisches Signal einer auf der jeweiligen Sensor-Einrichtung angeordneten Nervenzelle) korrelierte Ausgangsspannung aus. Um diesen Sachverhalt schematisch darzustellen, sind die Sensor-Einrichtungen 101 bis 104 schematisch als Spannungsquellen $V_1$, $V_2$, $V_3$, $V_4$ dargestellt.

Gemäß dem in Fig.1 dargestellten Szenario ist die erste Sensor-Einrichtung 101 über den Multiplexer 105 mit der Auslese-Schaltung 106 gekoppelt. Mittels der Auslese-Schaltung 106 wird die Ausgangsspannung einer jeweiligen Sensor-Einrichtung 101 bis 104 für eine Weiterverarbeitung verstärkt.

Ist die Aufnahme der Messwerte einer Vielzahl von Sensor-Einrichtungen mit einer hohen Abtastrate erforderlich, so muss eine einzelne Sensor-Einrichtung in einer sehr kurzen Zeit ausgelesen werden. Daraus ergibt sich eine hohe Anforderung an die Zeitauflösung des Systems.

Die in Fig.2 gezeigte Sensor-Anordnung 200 gemäß dem Stand der Technik zeigt neben den in Fig.1 beschriebenen Komponenten einen Innenwiderstand $R_i$ 201 jeder der Sensor-Einrichtungen sowie eine gemeinsame Kapazität C 202 der Sensor-Einrichtungen 101 bis 104. Die Zeitkonstante der Sensor-Anordnung 200 ist über das RC-Glied aus Innenwiderstand $R_i$ der Sensor-Einrichtungen 101 bis 104 sowie der umzuladenden Kapazität C 202 bestimmt. Diese beiden Parameter unterliegen für eine bestimmte Fertigungstechnologie bestimmten Beschränkungen. Somit ist auch die erzielbare Zeitkonstante $\tau$=RC beschränkt.

Die Zeitkonstante $\tau$ gibt anschaulich die Zeit an, nach der das Ausgangssignal bis auf (1-1/e)=63% auf den Endwert angestiegen ist. Sollen durch das Einschwingverhalten der Sensor-Anordnung 200 bedingte Messfehler unterhalb einer bestimmten, gerade noch tolerierbaren Grenze liegen, müssen unter Umständen Vielfache dieser Zeitkonstanten (z.B $2\tau$ oder $3\tau$) abgewartet werden. Somit ist die Zeitkonstante $\tau$=RC ein charakteristisches Maß für die minimal erreichbare Zeit zwischen zwei aufeinanderfolgenden Messungen. Daher resultiert aus dem Wert von $\tau$ eine Beschränkung der maximalen Anzahl von Sensor-Einrichtungen, die innerhalb eines vorgegebenen Zeitabschnitts bei einer vorgegebenen Abtastrate ausgelesen werden können.

Fig.3 ist ein Diagramm 300, in dem schematisch die Zeitabhängigkeit des Signalverlaufs an einer Sensor-Einrichtung 201 bis 204 nach dem Herstellen der Kopplung (t=0) der jeweiligen Sensor-Einrichtung mit der Auslese-Schaltung 106 über den Multiplexer 105 gezeigt ist.

Entlang der Abszisse 301 des Diagramms 300 ist die Zeit aufgetragen, die seit dem Umschalten des Multiplexers 105 (t=0) vergangen ist. Zum Zeitpunkt $t_0$ ist der Signalverlauf erstmals unter den vorgegebenen tolerierbaren Wert $V_{tol}$ gefallen. Entlang der Ordinate 302 des Diagramms 300 ist der zeitliche Signalverlauf an den in Fig.1, Fig.2 schematisch dargestellten Spannungsquellen $V_1$ bis $V_4$ dargestellt. Die Signalverlauf-Kurve 303 spiegelt das Einschwingverhalten an der Kapazität 202 bzw. den Widerständen $R_i$ wider, und ist guter Näherung eine abfallende Exponentialfunktion. Anschaulich ist zum Zeitpunkt $t_0$ erstmals der dynamische Fehler unter den tolerierbaren Fehler abgefallen, so dass die mindestens erforderliche Messzeit $t_0$ ist.

Bei aus dem Stand der Technik bekannten Sensor-Anordnungen werden die Sensor-Einrichtungen eines Sensor-Arrays im Spannungsbereich ausgelesen, das heißt, dass die Messgröße in eine elektrische Spannung umgesetzt wird. Um die RC-Konstante gering zu halten, und daher ein ausreichend schnelles Auslesen ausreichend vieler Sensor-Einrichtungen zu ermöglichen, wird versucht, den Innenwiderstand $R_i$ und als Folge davon die Zeitkonstante $\tau$ zu reduzieren.

Eine Möglichkeit, den Innenwiderstand $R_i$ der Sensor-Einrichtungen unabhängig von der Schaltungsarchitektur zu verringern, ist die Erhöhung der Treiberfähigkeit der Ausgangstransistoren einer Schaltung. Der Nachteil dieser Maßnahme ist ein erhöhter Flächenbedarf und eine stärkere kapazitive Belastung der vorangehenden Verstärkerstufe bzw. des angeschlossenen Sensors, was zu einer Abschwächung des Signals führt. Aufgrund der häufig starken Flächenbeschränkung und des Bedarfs an einer effizienten Flächennutzung ist diese Lösung für viele Anwendungsgebiete nicht geeignet.

Üblicherweise werden für integrierte Schaltkreise, die im Spannungsbereich betrieben werden, eine Reihe von Grundschaltungen für die Ausgangstreiber verwendet. Nachteilig an einer zusätzlichen, in die Sensor-Einrichtungen integrierten Ausgangstreiber-Schaltung ist die Erhöhung des Platzbedarfs einer Sensor-Einrichtung und somit die Verringerung der erzielbaren räumlichen Auflösung. Rahmenbedingung für die folgende Betrachtung ist, dass ein mit einer Sensor-Elektrode gekoppelter Ausgangstransistor simultan zum Signalverstärken verwendet wird. Dies ist jedoch keine zwingende Voraussetzung.

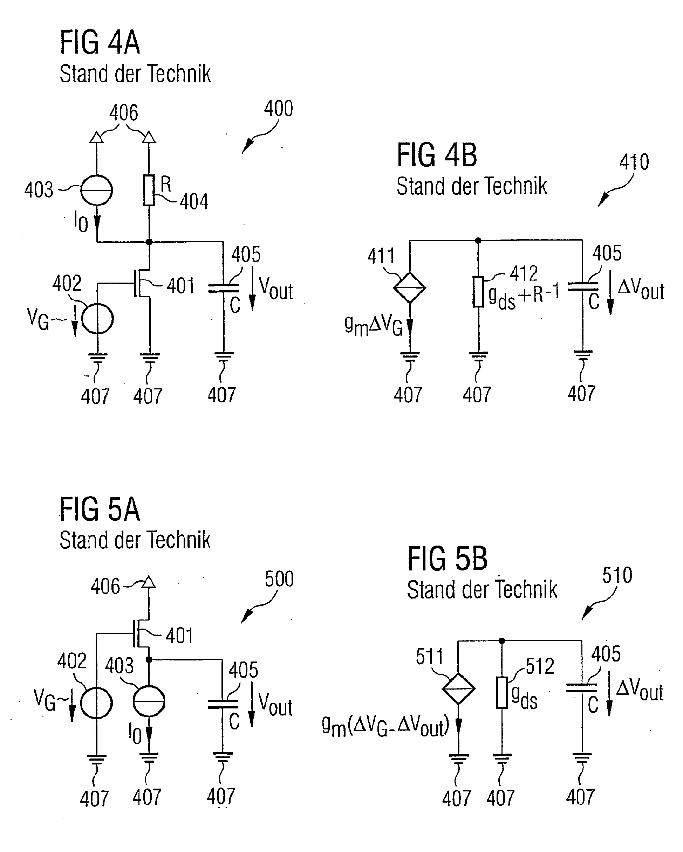

In Fig.4A ist eine Ausgangstreiber-Schaltung 400 dargestellt, bei der ein MOS-Transistor 401 in Source-Schaltung betrieben ist.

Die Sensor-Einrichtung ist als Spannungsquelle $V_G$ 402 dargestellt. Ferner ist bei der Ausgangstreiber-Schaltung 400 eine Konstantstromquelle $I_0$ 403, ein den Innenwiderstand der Sensor-Einrichtung repräsentierender ohmscher Widerstand R 404, sowie ein die Kapazität einer Sensor-Anordnung repräsentierende Kapazität C 405 gezeigt. An der Kapazität 405 liegt die Ausgangsspannung $V_{out}$ an. Die Spannungsquelle 402 ist mit dem Gate-Anschluss eines MOS-Transistors 401 gekoppelt. Der eine Source-/Drain-Anschluss des MOS-Transistors 401 ist auf Massepotential 407, wohingegen der andere Source-/Drain-Anschluss des MOS-Transistors 401 sowohl mit der Konstantstromquelle 403 als auch mit dem ohmschen Widerstand 404 und mit der Kapazität 405 gekoppelt ist. Der Arbeitspunkt des MOS-Transistors 401 ist mittels der Konstantstromquelle 403, des ohmschen Widerstands 404 und der Spannungsquelle $V_G$ 402 bestimmt.

In **Fig.4B** ist ein Kleinsignalersatzschaltbild 410 der in Fig.4A gezeigten Ausgangstreiber-Schaltung 400 gezeigt. In dem Ersatzschaltbild 410 ist eine gesteuerte Stromquelle 411 ($g_m \Delta V_G$), ein effektiver Innenwiderstand 412 ($g_{ds}+R^{-1})^{-1}$ sowie die umzuladende Kapazität 405 gezeigt. Mit $g_{ds}$ ist der Ausgangsleitwert des MOS-Transistors 401 bezeichnet, $g_m$ ist die Steilheit des MOS-Transistors 401.

Bei der in Fig.4A, Fig.4B gezeigten Source-Schaltung des MOS-Transistors 401 ist der eine Source-/Drain-Anschluss auf Massepotential 407. Der Arbeitspunkt der Ausgangstreiber-Schaltung 400 ist über den Gleichanteil der Spannungsquelle $V_G$ 402 am Gate-Anschluss des MOS-Transistors 401 und über die mit dem anderen Source-/Drain-Anschluss des MOS-Transistors 401 gekoppelte Konstantstromquelle $I_0$ mit dem Innenwiderstand R vorgegeben. Alternativ ist auch eine Lösung ohne Konstantstromquelle $I_0$ 403, nur mit einem ohmschen Widerstand R 404 möglich. Bei der in Fig.4B gezeigten Ausgangstreiber-Schaltung 400 ist an jeweils einen Anschluss der Konstantstromquelle 403 und des ohmschen Widerstands 404 eine Versorgungsspannung 406 angelegt, wohingegen ein Anschluss der Spannungsquelle 402, der eine Source-/Drain-Anschluss des MOS-Transistors 401 und ein Anschluss der Kapazität 405 auf Massepotential 407 sind.

Wird die Gate-Spannung $V_G$ moduliert (beispielsweise infolge eines Sensor-Ereignisses), so ändert sich der Drainstrom $I_D$ des MOS-Transistors 401 um $g_m \Delta V_G$. Dies ist mittels der gesteuerten Stromquelle 411 aus Fig.4B symbolisiert, in der das Kleinsignalersatzschaltbild der Ausgangstreiber-Schaltung 400 gezeigt ist. Die Änderung der Ausgangsspannung $\Delta V_{out}$ resultiert aus dem geänderten Spannungsabfall an dem MOS-Transistor 401 und an dem effektiven Widerstand 412. Für niedrige Frequenzen $\omega$ kann die Kapazität C in guter Näherung vernachlässigt werden, hieraus resultiert eine Leerlaufverstärkung $A(\omega=0)$, die mittels des folgenden Ausdrucks beschreibbar ist:

$$A(\omega=0) = \Delta V_{out}/\Delta V_G = g_m/(g_{ds}+R^{-1}) \qquad (1)$$

Setzt man in Gleichung (1) Transistor-Parameter ein, die für die CMOS-Technologie typisch sind, resultiert hieraus eine mögliche Spannungsverstärkung um einen Faktor von ungefähr zehn bis ungefähr fünfzig. Dadurch werden auch Signale kleiner Amplitude nach der ersten Verstärkerstufe so verstärkt, dass sie durch Rauscheffekte nicht mehr nennenswert gestört werden. Die Zeitkonstante $\tau_C$ des Verstärkers ergibt sich zu:

$$\tau_C = C/(g_{ds}+R^{-1}) \qquad (2)$$

Die Kapazität ist durch die verwendete Technologie vorgegeben und kann insofern nur in sehr eingeschränktem Maße beeinflusst werden, beispielsweise mittels einer Optimierung des Layouts. Die Parameter R und $g_{ds}$ können für eine bestimmte Verstärkung und aufgrund von Flächenbeschränkungen nicht beliebig variiert werden, so dass aus dieser Randbedingungen ein Grenzwert für die maximal übertragbare Bandbreite resultiert.

Im Weiteren wird bezugnehmend auf Fig.5A eine weitere Ausgangstreiber-Schaltung 500 gemäß dem Stand der Technik beschrieben. Bezugnehmend auf Fig.5B wird ein Ersatzschaltbild 510 der in Fig.5A gezeigten Ausgangstreiber-Schaltung 500 beschrieben. Diejenigen Komponenten der Ausgangstreiber-Schaltung 500, die auch in der Ausgangstreiber-Schaltung 400 enthalten sind, sind mit den gleichen Bezugsziffern versehen.

Bei der in Fig.5A gezeigten Ausgangstreiber-Schaltung 500 ist der MOS-Transistor 401 in Source-Folger-Schaltung ausgestaltet. Abweichend von der in Fig.4A gezeigten Ausgangstreiber-Schaltung 400 ist derjenige Source-/Drain-Anschluss des MOS-Transistors 401, der nicht mit der Konstantstromquelle 403, der Spannungsquelle 402 und der Kapazität 405 gekoppelt ist, auf dem Potential der Versorgungsspannung 406. Ferner sind bei der Ausgangstreiber-Schaltung 500 ein Anschluss der Konstantstromquelle 403 und der Kapazität 405 auf dem elektrischen Massepotential 407. Die Konstantstromquelle $I_0$ 403 und die Spannungsquelle $V_G$ 402 bestimmen den Arbeitspunkt des MOS-Transistors 401.

Der elektrische Strom an dem mit der Konstantstromquelle $I_0$ 403 gekoppelten Source-/Drain-Anschluss des MOS-Transistors 401 ist mittels der Konstantstromquelle $I_0$ 403 bestimmt. Die Gate-Spannung des MOS-Transistors 401 ist mittels der Spannungsquelle $V_G$ 402 eingestellt. Ferner ist eine umzuladende Kapazität C 405 gezeigt.

In dem Kleinsignalersatzschaltbild 510 von **Fig.5B** ist eine gesteuerte Stromquelle $g_m(\Delta V_G - \Delta V_{out})$ 511 sowie ein Innenwiderstand $g_{ds}$ gezeigt. Gemäß dem in Fig.5B gezeigten Kleinsignal-Ersatzschaltbild 510 ist die auf dem Feldeffekt beruhende Modulation des elektrischen Stroms an dem gemäß Fig.5A unteren Source-/Drain-Anschluss des MOS-Transistors 401 als gesteuerte Stromquelle $g_m(\Delta V_G - V_{out})$ 511 dargestellt.

Die Abhängigkeit dieses elektrischen Stromes von der elektrischen Spannung an dem gemäß Fig.5A unteren Source-/Drain-Anschluss des MOS-Transistors 401 ist durch den Leitwert $g_{ds}$ bestimmt. Die Zeitkonstante $\tau_C$ des Verstärkers ist von dem RC-Glied bestimmt und wird durch den folgenden Ausdruck beschrieben:

$$\tau_C = C/(g_{ds}+g_m) \qquad (3)$$

Die Zeitkonstante $\tau_C$ aus Gleichung (3) entspricht dem Szenario der Source-Schaltung des MOS-Transistors 401 aus Fig.4A, falls der Wert des ohmschen Widerstands R gleich der inversen Steilheit $g_m^{-1}$ des MOS-Transistors 401 ist (vgl.

Gleichung (2)). Gemäß beiden Schaltungsarchitekturen ist die Verstärkung dann näherungsweise eins, nämlich:

$$A(\omega=0) = \Delta V_{out}/\Delta V_G = g_m/(g_m+g_{ds}) \leq 1 \qquad (4)$$

Diese niedrige Verstärkung ist aufgrund der kleinen Signalamplituden, die aus der Sensor-Einrichtung herausgeleitet werden müssen, von Nachteil, da Rauscheffekte das gemessene Signal verfälschen können.

Als Beispiel für eine Verwirklichung des beschriebenen Prinzips anhand einer CMOS-Kamera sei auf [1] verwiesen.

In [2] wird ein System beschrieben, das einen Biosensor verwendet, bei dem unter Verwendung eines Feldeffekttransistors organische Verbindungen analysiert werden.

In [3] wird eine Vorrichtung zum Detektieren von Molekülen beschrieben, wobei zur Erkennung der Moleküle Transistoren verwendet werden.

In [4] wird eine Gas-Detektions-Vorrichtung beschrieben, die als Detektionselement einen Transistor aufweist.

In [5] wird eine Sensor-Anordnung mit chemisch sensitiven JFET-Transistoren beschrieben.

In [6] wird eine Vorrichtung zum Messen der Konzentration von Bestandteilen in Fluiden beschrieben, wobei zu dieser Messung ein chemisch sensitiver Feldeffektransistor verwendet wird.

In [7] werden Sensoren mit chemisch sensitiven Feldeffekttransistoren beschrieben, die Membranen auf der Gate-Isolationsschicht aufweisen.

Zusammenfassend ist die Funktionalität der aus dem Stand der Technik bekannten Schaltungsarchitekturen zum Auslesen von Sensor-Signalen eines Sensor-Arrays ungenügend, da aus der umzuladenden Kapazität eine große Zeitkonstante zum Auslesen der einzelnen Sensor-Einrichtungen resultiert. Dies führt zu einer schlechten Zeitauflösung. Ferner ist die Verstärkung des häufig kleinen Sensor-Signals bei aus dem Stand der Technik bekannten Schaltungsarchitekturen oft nicht ausreichend.

Der Erfindung liegt das Problem zugrunde, eine Sensor-Anordnung mit hoher räumlicher Auflösung zu schaffen, die bei einer ausreichend hohen Signalverstärkung ein schnelles Auslesen von Sensor-Signalen, d.h. eine hohe Bandbreite, ermöglicht und die unabhängig von Parameterschwankungen ihrer elektronischen Bauelemente ist.

Das Problem wird durch eine Sensor-Anordnung und durch ein Verfahren zum Betreiben einer Sensor-Anordnung mit den Merkmalen gemäß den unabhängigen Patentansprüchen gelöst.

Die erfindungsgemäße Sensor-Anordnung enthält eine Mehrzahl von auf und/oder in einem Substrat ausgebildeten Sensor-Einrichtungen. Jede Sensor-Einrichtung hat einen elektrischen Signal-Umsetzer und ein mit dem Signal-Umsetzer gekoppeltes Sensor-Element, mit dem die elektrische Leitfähigkeit des Signal-Umsetzers infolge eines Sensor-Ereignisses auf dem Sensor-Element charakteristisch beeinflussbar ist. Ferner weist die erfindungsgemäße Sensor-Einrichtung eine Einrichtung zum Konstanthalten einer an dem Signal-Umsetzer anliegenden elektrischen Spannung auf. Darüber hinaus hat jede Sensor-Einrichtung eine Einrichtung zum Erfassen des Werts des durch den Signal-Umsetzer fließenden elektrischen Stromes als Sensor-Signal. Bei dem elektrischen Signal-Umsetzer handelt es sich um einen Feldeffekttransistor, dessen Gate-Anschluss mit dem Sensor-Element gekoppelt ist, wobei die Einrichtung zum Konstanthalten einer elektrischen Spannung derart eingerichtet ist, dass sie die elektrische Spannung zwischen den Source-/Drain-Anschlüssen des Feldeffekttransistors konstant hält. Ferner weist die Sensor-Anordnung eine Kalibrier-Einrichtung einer jeweiligen Sensor-Einrichtung auf, die derart eingerichtet ist, dass mit ihr der Gate-Bereich des Feldeffekttransistors auf ein derartiges elektrisches Kalibrier-Potential bringbar ist, dass der elektrische Strom von Parameterschwankungen des Feldeffekttransistors unabhängig ist.

Ferner ist erfindungsgemäß ein Verfahren zum Betreiben einer Sensor-Anordnung mit den oben genannten Merkmalen bereitgestellt, wobei gemäß dem Verfahren die elektrische Leitfähigkeit des Signal-Umsetzers infolge eines Sensor-Ereignisses auf einem jeweiligen Sensor-Element charakteristisch beeinflusst wird. Ferner wird die elektrische Spannung an dem Signal-Umsetzer konstant gehalten. Der durch den Signal-Umsetzer fließende elektrische Strom wird als Sensor-Signal erfasst. Es wird zumindest ein Teil der Sensor-Einrichtungen kalibriert, indem der Gate-Bereich des jeweiligen Feldeffekttransistors auf ein derartiges elektrisches Kalibrier-Potential gebracht wird, dass der Wert des elektrischen Stroms bei einem Sensor-Ereignis von Parameterschwankungen des Feldeffekttransistors unabhängig ist.

Eine Grundidee der Erfindung beruht darauf, anstelle der elektrischen Spannung einen elektrischen Strom an einem mit dem Sensor-Element gekoppelten Signal-Umsetzer zu erfassen. Indem erfindungsgemäß ein elektrischer Strom und nicht, wie gemäß dem Stand der Technik eine elektrische Spannung, erfasst wird, ist ein Umladen von Kapazitäten vermieden. Dadurch ist eine größere Bandbreite, das heißt ein schnelleres Auslesen der Sensor-Elemente einer Sensor-Anordnung, ermöglicht. Die Zeitkonstante des Systems ist nicht mehr durch die Verschaltung innerhalb der Sensor-Einrichtung festgelegt, sondern nur noch durch die externe Schaltung. Anschaulich ist erfindungsgemäß die elektrische Spannung als Sensor-Signal extern stabilisiert. Mit der erfindungsgemäßen Schaltungsarchitektur für eine Sensor-Anordnung ist bei einer vorgegebenen Technologie eine besonders hohe Anzahl von Sensor-Elementen und eine besonders hohe Abtastrate ermöglicht, wodurch eine kleine Zeitkonstanten zum Auslesen der Sensor-Elemente erreicht ist. Aufgrund der Verschaltung der erfindungsgemäßen Sensor-Einrichtung wird ein elektrischer Sensor-Strom statt einer elektrischen Sensor-Spannung erfasst, was zu einer hohen Verstärkung und einer kleinen Zeitverzögerung führt.

Wird anstelle der elektrischen Spannung $V_{out}$ der elektrische Strom $I_{out}$ als Ausgangsgröße der Ausgangsstufe einer Sensor-Einrichtung bzw. des Signal-Umsetzers verwendet, so wird die Spannung an der Kapazität unabhängig von der Ausgangsgröße und kann zum Beispiel konstant auf einem Wert der Ausgangsspannung gehalten werden. Da die induktiven Eigenschaften von integrierten Zuleitungen gegenüber den kapazitiven Eigenschaften in der Regel zu vernachlässigen sind, resultiert daraus eine erheblich verringerte Zeitkonstante. In der Praxis führt ein unvermeidlicher Innenwiderstand der Messschaltung zu einem geringen Spannungsabfall. Da sich jedoch die Messschaltung außerhalb der Sensor-Einrichtungen befindet, kann ihr Innenwiderstand gering gehalten werden. Dadurch ist die Zeitkonstante $\tau=RC$ um Größenordnungen kleiner als gemäß dem Stand der Technik. Dadurch sind schneller auslesbare Sensor-Anordnungen bzw. eine erhöhte Anzahl von Sensor-Einrichtungen ermöglicht.

Anschaulich wird eine Ausgangstreiber-Schaltung einer Sensor-Einrichtung in einer "Current-Mode-Technik" ausgeführt.

Ein weiterer wichtiger Aspekt der Erfindung ist darin zu sehen, dass eine Mehrzahl von Sensor-Einrichtungen auf und/oder in dem Substrat ausgebildet sind. Mit anderen Worten ist die erfindungsgemäße Sensor-Anordnung als integrierter Schaltkreis, beispielsweise in und/oder auf einem Silizium-Substrat (z.B. Wafer, Chip, etc.) realisiert. Dadurch ist eine Miniaturisierung erreicht und somit ein Array mit einer hohen Anzahl von Sensor-Einrichtungen geschaffen. Ferner kann die Sensor-Anordnung mit vertretbarem Aufwand unter **Verwendung** der modernen und ausgereiften Silizium-Mikrotechnologie herstellt werden.

Bevorzugte Weiterbildungen der Erfindung ergeben sich aus den abhängigen Ansprüchen.

Bei der erfindungsgemäßen Sensor-Einrichtungen ist der elektronische Signal-Umsetzer ein Feldeffekttransistor, dessen Gate-Anschluss mit dem Sensor-Element gekoppelt ist, wobei die Einrichtung zum Konstanthalten einer elektrischen Spannung derart eingerichtet ist, dass sie die elektrische Spannung zwischen den Source-/Drain-Anschlüssen des Feldeffekttransistors konstant hält. Ein Feldeffekttransistor als Signal-Umsetzer weist die Funktionalität auf, dass eine Modulation der Gate-Spannung in eine veränderte elektrische Leitfähigkeit des Kanal-Bereichs des Feldeffekttransistors umgesetzt wird, so dass der Wert des durch einen Source-/Drain-Anschluss fließenden elektrischen Stroms aufgrund des veränderten ohmschen Widerstands des Kanal-Bereichs des Feldeffekttransistors charakteristisch beeinflusst ist.

Ferner kann die erfindungsgemäße Sensor-Einrichtung eine Auswerte-Einheit aufweisen, wobei der Auswerte-Einheit der Wert des elektrischen Stroms als Sensor-Signal bereitgestellt ist.

Die Auswerte-Einheit ist vorzugsweise derart eingerichtet, dass sie aus dem Wert des elektrischen Stroms eine für diesen Wert charakteristische elektrische Spannung bildet oder den Wert des elektrischen Stroms auf einen diesen charakterisierenden digital kodierten Wert abbildet.

Mit anderen Worten kann der erfasste elektrische Strom in eine elektrische Spannung umgewandelt werden, was für die Weiterverarbeitung des Signals vorteilhaft sein kann. Ferner kann ein analoges Stromsignal in ein digitales und somit fehlerrobusteres Signal umgewandelt werden.

Insbesondere kann die Auswerte-Einheit einen Operationsverstärker, insbesondere in einer Verschaltung als Spannungsfolger, aufweisen, der einen ersten Eingang aufweist, an den das Sensor-Signal anlegbar ist. Ferner weist der Operationsverstärker einen zweiten Eingang auf, an den ein elektrisches Referenz-Potential anlegbar ist. An einem Ausgang des Operationsverstärkers ist die charakteristische elektrische Spannung bereitgestellt, wobei der erste Eingang und der Ausgang mittels eines ohmschen Widerstands miteinander gekoppelt sind.

Die Sensor-Anordnung kann als Biosensor-Anordnung ausgestaltet sein. Das Sensor-Element einer jeden Sensor-Einrichtung kann beispielsweise ein elektrisches Signal einer auf dem Sensor-Element aufgewachsenen Nervenzelle erfassen. Alternativ kann das Sensor-Element unter Verwendung eines ISFETs elektrisch geladene Partikel auf dem Sensor-Element detektieren.

Die Sensor-Anordnung weist eine Kalibrier-Einrichtung zum Kalibrieren einer jeweiligen Sensor-Einrichtung auf, die derart eingerichtet ist, dass mit ihr der Gate-Bereich des Feldeffekttransistors auf ein derartiges elektrisches Kalibrier-Potential bringbar ist, dass der elektrische Strom von Parameterschwankungen des Feldeffekttransistors unabhängig ist. Beispielsweise können bedingt durch ein Herstellungsverfahren Feldeffekttransistoren unterschiedlicher Sensor-Einrichtungen unterschiedliche Parameter (z.B. Schwellenspannung) aufweisen. Mittels der Kalibrierung kann sichergestellt werden, dass Parameterschwankungen nicht zu einer Verfälschung beim Erfassen eines Sensor-Ereignisses führen.

Insbesondere kann mittels Implementierens einer Kalibrier-Einrichtung basierend auf der Auto-Zeroing-Technik die Sensor-Einrichtung kalibriert werden, so dass eine robuste Sensor-Anordnung geschaffen ist. Eine Signal-Beeinflussung aufgrund von Parameter-Schwankungen der Komponenten einer jeweiligen Sensor-Einrichtung, beispielsweise der als Feldeffekttransistoren realisierten Signal-Umsetzer, ist dadurch vermieden.

Die Kalibrier-Einrichtung kann derart eingerichtet sein, dass zum Kalibrieren an den Gate-Anschluss und an einen Source-/Drain-Anschluss des Feldeffekttransistors ein elektrischer Kalibrierstrom anlegbar ist.

Alternativ kann die Auswerte-Einheit eine Correlated-Double-Sampling-Einrichtung aufweisen, die derart eingerichtet ist, dass sie bei einem Sensor-Ereignis einen von Parameterschwankungen eines jeweiligen Feldeffekttransistors unabhängigen Wert des elektrischen Stroms bildet.

Gemäß dem Correlated-Double-Sampling-Prinzip wird anschaulich in einem ersten Schritt ein Sensor-Ereignis in einer Sensor-Einrichtung detektiert und das Sensor-Signal abgespeichert.

Dieses Sensor-Signal ist von der Veränderung des Werts der physikalischen Parameter der Sensor-Einrichtung (beispielsweise geometrischer Parameter eines Feldeffekttransistors) abhängig und kann ferner von physikalischen Parametern weiterer Komponenten, beispielsweise eines Verstärkers zum Verstärken des Sensor-Signals, abhängig sein. In einem zweiten Schritt wird in Abwesenheit eines Sensor-Ereignisses ein Hilfssignal erfasst, das nur von dem Wert des physikalischen Parameters der Sensor-Einrichtung abhängt. Subtrahiert man das Hilfssignal von dem Sensor-Signal, so erhält man ein von dem Wert des physikalischen Parameters im Wesentlichen unabhängiges Sensor-Signal.

Insbesondere kann die Correlated-Double-Sampling-Einrichtung der Erfindung derart eingerichtet sein, dass mit ihr in einer Kalibrierphase der Gate-Bereich des Feldeffekttransistors auf ein elektrisches Kalibrier-Potential gebracht wird, und der zugehörige Wert des elektrischen Stroms als Kalibrier-Signal erfasst und gespeichert wird. In einer Erfassungsphase kann der Wert der elektrischen Stroms infolge eines Sensor-Ereignisses als Sensor-Signal erfasst werden.

In einer Auswertephase können Sensor-Signal und Kalibrier-Signal gemeinsam ausgewertet werden.

Vorzugsweise sind die Sensor-Einrichtungen der Sensor-Anordnung im Wesentlichen matrixförmig in und/oder auf dem **Substrat** angeordnet, und sind mittels Zeilen- und Spaltenleitungen derart miteinander verschaltet, dass die Sensor-Einrichtungen einzeln, zeilenweise bzw. spaltenweise ansteuerbar sind.

Bei der erfindungsgemäßen Sensor-Anordnung kann mindestens eine Auswerte-Einheit, mindestens eine Kalibrier-Einrichtung und/oder mindestens eine Correlated-Double-Sampling-Einrichtung für zumindest einen Teil der Sensor-Einrichtungen einer Zeilenleitung bzw. einer Spaltenleitung gemeinsam vorgesehen sein.

Im Weiteren wird das erfindungsgemäße Verfahren zum Betreiben der erfindungsgemäßen Sensor-Anordnung näher beschrieben. Ausgestaltungen der Sensor-Anordnung gelten auch für das Verfahren zum Betreiben der Sensor-Anordnung und umgekehrt.

Gemäß dem erfindungsgemäßen Verfahren kann als elektronischer Signal-Umsetzer einer jeweiligen Sensor-Anordnung ein Feldeffekttransistor verwendet werden, dessen Gate-Anschluss mit dem Sensor-Element gekoppelt wird, wobei mittels der Einrichtung zum Konstanthalten einer elektrischen Spannung die elektrische Spannung zwischen den Source-/Drain-Anschlüssen des Feldeffekttransistors konstant gehalten wird.

Ferner kann eine jeweilige Sensor-Einrichtung kalibriert werden, indem der Gate-Bereich des Feldeffekttransistors auf ein derartiges elektrisches Kalibrier-Potential gebracht wird, dass der Wert des elektrischen Stroms bei einem Sensor-Ereignis von den Eigenschaften des Feldeffekttransistors (z.B. einer fertigungsbedingte Abweichung der Dicke der Gateisolierenden Schicht von einem Soll- oder Mittel-Wert) unabhängig wird.

Unter Verwendung des Correlated-Double-Sampling-Verfahrens kann bei einem Sensor-Ereignis ein von den Eigenschaften des Feldeffekttransistors unabhängiger Wert des elektrischen Stroms gebildet werden.

Ausführungsbeispiele der Erfindung sind in den Figuren dargestellt und werden im Weiteren näher erläutert.

Es zeigen:

Figur 1         eine Darstellung einer Sensor-Anordnung gemäß dem Stand der Technik,

Figur 2         eine Darstellung einer anderen Sensor-Anordnung gemäß dem Stand der Technik,

Figur 3         ein Diagramm, in dem ein Signalverlauf in Abhängigkeit von einer Auslesezeit dargestellt ist,

Figur 4A, Figur 4B     eine Ausgangstreiber-Schaltung und ein zugehöriges Kleinsignalersatzschaltbild gemäß dem Stand der Technik,

Figur 5A, Figur 5B     eine andere Ausgangstreiber-Schaltung und ein zugehöriges Kleinsignalersatzschaltbild gemäß dem Stand der Technik,

Figur 6         eine Sensor-Einrichtung gemäß einem bevorzugten Ausführungsbeispiel der Erfindung,

Figur 7         eine Auswerte-Einheit gemäß der Erfindung,

Figur 8         eine Sensor-Anordnung gemäß ersten Ausführungsbeispiel der Erfindung,

Figur 9         eine Sensor-Anordnung gemäß einem zweiten Ausführungsbeispiel de Erfindung,

Figur 10        eine Sensor-Anordnung gemäß einem dritten Ausführungsbeispiel der Erfindung.

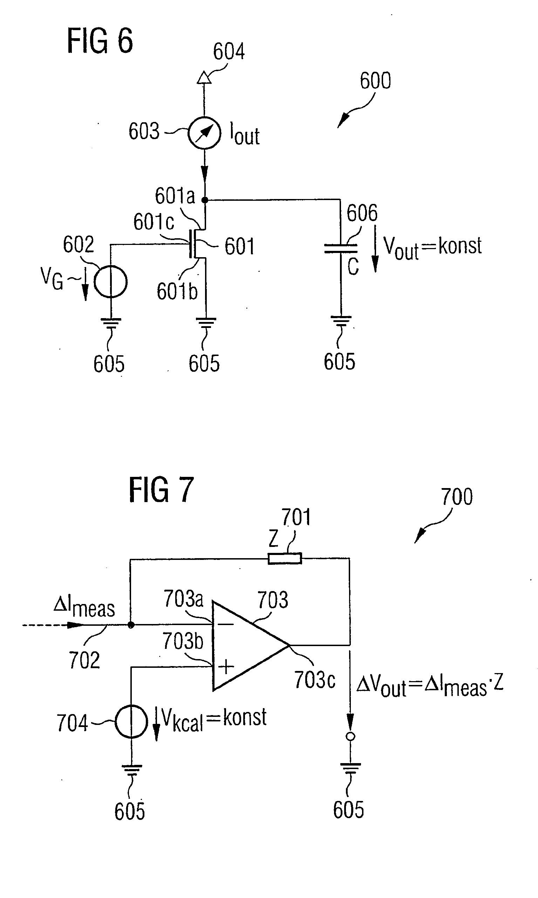

Im Weiteren wird bezugnehmend auf Fig.6 eine Sensor-Einrichtung 600 beschrieben.

Die in Fig.6 gezeigte Sensor-Einrichtung 600 weist einen Feldeffekttransistor 601 als elektrischen Signal-Umsetzer auf. Ferner weist die Sensor-Einrichtung 600 ein mit dem Feldeffekttransistor 601 gekoppeltes Biosensor-Element auf, das in Fig.6 schematisch als Spannungsquelle 602 dargestellt ist. Erfolgt an dem Biosensor-Element ein Sensor-Ereignis, so wird infolge dieses Sensor-Ereignisses die elektrische Leitfähigkeit des Kanal-Bereichs des Feldeffekttransistors 601 charakteristisch beeinflusst.

Ein erster Source-/Drain-Anschluss 601a des Feldeffekttransistors 601 ist mit einem Anschluss eines Amperemeters 603 zum Erfassen eines Sensorstroms $I_{out}$ gekoppelt, an dessen anderen Anschluss eine Versorgungsspannung 604 angelegt ist. Ein zweiter Source-/Drain-Anschluss 601b des Feldeffekttransistors 601 ist auf dem Massepotential 605. Aufgrund dieser Verschaltung ist zwischen den Source-/Drain-Anschlüssen 601a, 601b eine konstante elektrische Potentialdifferenz angelegt. Ferner liegt auch an einem Kondensator 606, der die effektive Kapazität der Sensor-Einrichtung 600 repräsentiert, die konstante Spannung $V_{out}$ an, die sich aus dem Differenz zwischen der Versorgungsspannung 604 und dem Massepotential 605 ergibt. Das Amperemeter 603 ist eine Einrichtung zum Erfassen des Werts des durch den ersten Source-/Drain-Anschluss 601a des Feldeffekttransistors 601 fließenden elektrischen Stroms als Sensor-Signal.

Der Gate-Anschluss 601c des Feldeffekttransistors 601 ist mit der Spannungsquelle 602 gekoppelt. Die elektrische Spannung zwischen den Source-/Drain-Anschlüssen 601a, 601b des Feldeffekttransistors 601 ist konstant. Der erste Source-/Drain-Anschluss 601a des Feldeffekttransistors 601 ist mit einem Anschluss des Kondensators 606 gekoppelt und ist ferner mit dem Amperemeter 603 gekoppelt.

Erfolgt auf dem Biosensor-Element ein Sensor-Ereignis, so wird dadurch die elektrische Spannung $V_G$ der Spannungsquelle 602 moduliert. Diese ist an dem Gate-Anschluss 601c des Feldeffekttransistors 601 bereitgestellt, so dass die elektrische Leitfähigkeit des Kanal-Bereichs des Feldeffekttransistors 601 dadurch charakteristisch beeinflusst wird. Dadurch ist der Wert des elektrischen Stromflusses durch den Source-/Drain-Anschluss 601a des Feldeffekttransistors 601, welcher Stromfluss von dem Amperemeter 603 erfasst wird, ein charakteristisches Maß für das Sensor-Ereignis.

Der Feldeffekttransistor 601 wird im "Current-Mode" betrieben. Die Spannungsquelle 602 $V_G$ bestimmt das an dem Gate-Bereich 601c anliegende Potential, wohingegen die Spannung an dem ersten Source-/Drain-Anschluss 601a fest auf der Versorgungsspannung 604 ist. Mittels des Amperemeters 603 wird der Ausgangsstrom $I_{out}$ erfasst. Es ist anzumerken, dass die Kapazität C 606 nicht umgeladen werden muss, da die Spannung konstant gehalten wird.

Im Weiteren wird bezugnehmend auf Fig.7 eine Auswerte-Einheit 700 gemäß einem bevorzugten Ausführungsbeispiel der Erfindung beschrieben.

Die Auswerte-Einheit hat die Funktionalität, die elektrische Spannung auf einer Sensor-Leitung konstant zu halten und simultan den erfassten Sensor-Strom (vorzugsweise linear) in eine Sensor-Spannung umzusetzen. Dies ermöglicht eine Weiterverarbeitung im Spannungsbereich.

Für manche Anwendungen kann es vorteilhaft sein, dass ein erfasster elektrischer Sensor-Strom in ein Spannungssignal umgewandelt wird. Die in Fig.7 gezeigte Auswerte-Einheit 700 zeigt ein Ausführungsbeispiel, mit dem es ermöglicht ist, ein Strom-Signal $\Delta I_{meas}$ in ein Spannungs-Signal $\Delta V_{out}$ umzuwandeln. Mittels der in Fig.7 gezeigten Schaltung wird sowohl die elektrische Spannung auf der Leitung konstant gehalten, als auch der modulierte Strom $I_{meas}$ in eine modulierte Ausgangsspannung $V_{out}=I_{meas}Z$ umgewandelt. Dabei steht Z für den Wert eines Widerstands 701.

Wie in Fig.7 gezeigt, wird an einem Eingang 702 der Auswerte-Einheit 700 der modulierte Wert des elektrischen Stroms $\Delta I_{meas}$ als Sensor-Signal bereitgestellt. Die Auswerte-Einheit 700 weist einen Operationsverstärker 703 auf. Der Operationsverstärker 703 hat einen nicht-invertierenden Eingang 703a, an dem das Sensor-Signal $\Delta I_{meas}$ bereitgestellt ist. Ferner weist der Operationsverstärker 703 einen nicht-invertierenden Eingang 703b auf, an den ein konstantes elektrisches Referenz-Potential $V_{kal}$ angelegt ist. An einem Ausgang 703c des Operationsverstärkers 703 ist die charakteristische elektrische Spannung $\Delta V_{out}$ bereitgestellt. Der Ausgang 703c des Operationsverstärkers 703 ist mit dem invertierenden Eingang 703a des Operationsverstärkers 703 über den Widerstand Z 701 rückgekoppelt. Wie ferner in Fig.7 gezeigt, ist das elektrische Potential $V_{kal}$ an dem nicht-invertierenden Eingang 703b des Operationsverstärkers 703 mittels der Konstantspannungsquelle 704 bereitgestellt. Die Konstantspannungsquelle 704 ist zwischen dem elektrischen Massepotential 605 und dem nicht-invertierenden Eingang 703b des Operationsverstärkers 703 geschaltet.

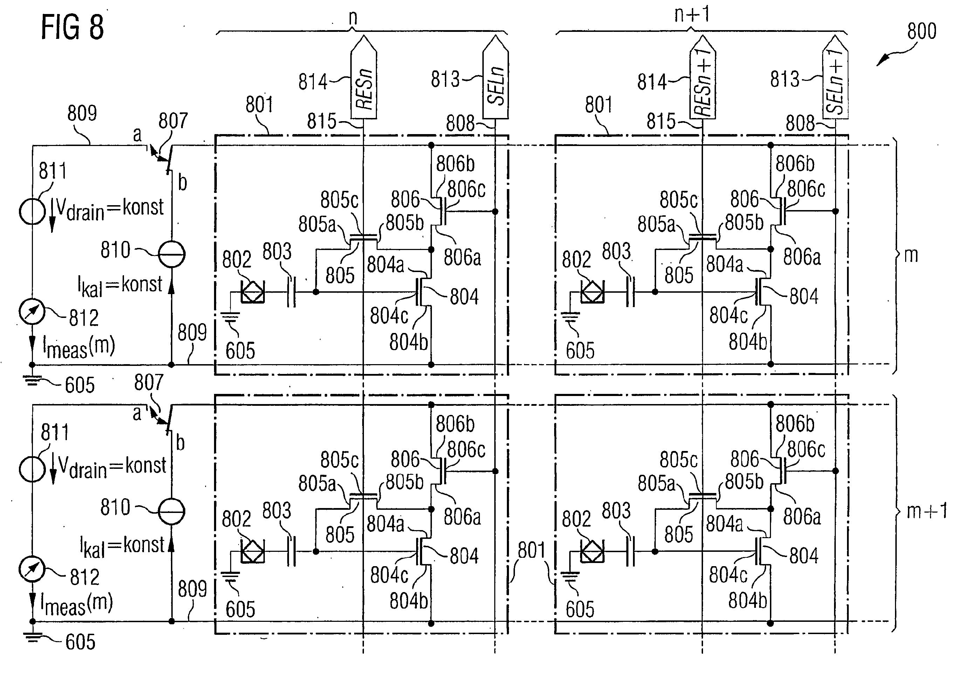

Im Weiteren wird bezugnehmend auf Fig.8 eine Sensor-Anordnung 800 gemäß einem ersten bevorzugten Ausführungsbeispiel der Erfindung beschrieben.

Die Sensor-Anordnung 800 weist eine Vielzahl von matrixförmig angeordneten Sensor-Einrichtungen 801 auf, die mittels Zeilen- und Spaltenleitungen derart verschaltet sind, dass die Sensor-Einrichtungen 801 einzeln bzw. spaltenweise ansteuerbar sind.

Die Sensor-Einrichtungen 801 der Sensor-Anordnung 800 sind auf und in einem Silizium-Substrat (nicht gezeigt) ausgebildet. Mit anderen Worten ist die Sensor-Anordnung 800 als integrierter Schaltkreis realisiert.

Obwohl die Sensor-Anordnung 800 eine Vielzahl von Sensor-Einrichtungen 801 aufweist, sind in Fig.8 zum Zwecke einer vereinfachten Darstellung nur die n-te und (n+1)-te Spalte von Sensor-Einrichtungen 801 sowie die m-te und (m+1)-te Zeile der Sensor-Einrichtungen 801 gezeigt.

Im Weiteren wird exemplarisch der Aufbau der in der n-ten Spalte und der m-ten Zeile angeordneten Sensor-Einrichtung 801 aus Fig.8 näher beschrieben.

Ein Sensor-Element 801 der Sensor-Anordnung 800 ist in Fig.8 schematisch als Spannungsquelle 802 gezeigt, das zwischen dem Massepotential 605 und einem Anschluss eines Kondensators 803 angeordnet ist. Der Kondensator 803 symbolisiert ein auf dem Sensor-Element aufgebrachtes Dielektrikum, das darauf befindliche, zu detektierende elektrisch geladene Partikel von dem Gate-Bereich 804c eines Erfass-Transistors 804 räumlich entkoppelt. Der andere Anschluss des Kondensators 803 ist ferner mit dem ersten Source-/Drain-Anschluss 805a eines Kalibrier-Transistors 805 gekoppelt. Der zweite Source-/Drain-Anschluss 805b des Kalibrier-Transistors 805 ist mit dem ersten Source-/Drain-Anschluss 804a des Erfass-Transistors 804 gekoppelt. Ferner ist der erste Source-/Drain-Anschluss 804a des Erfass-Transistors 804 mit dem ersten Source-/Drain-Anschluss 806a eines Auswahl-Transistors 806 gekoppelt. Der zweite Source-/Drain-Anschluss 806b des Auswahl-Transistors 806 ist mit einem Umschalt-Element 807 gekoppelt, das für jede Zeile von Sensor-Einrichtungen 801 gemeinsam vorgesehen ist. Der Gate-Anschluss 806c des Auswahl-Transistors 806 ist mit einer ersten Spaltenleitung 808 gekoppelt, die für jede Spalte von Sensor-Einrichtungen 801 gemeinsam vorgesehen ist. Der zweite Source-/Drain-Anschluss 804b des Erfass-Transistors 804 ist mit einer Zeilenleitung 809 gekoppelt, die für jede Zeile von Sensor-Einrichtungen 801 gemeinsam vorgesehen ist. Das Umschalt-Element 807 kann in einer von zwei Schaltstellungen "a" bzw. "b" geschaltet sein, je nachdem, ob ein Kalibrier-Modus oder ein Mess-Modus eingestellt werden soll. Gemäß dem in Fig.8 gezeigten Szenario ist das Schalterelement 807 in der Stellung "b", so dass der zweite Source-/Drain-Anschluss 806b des Auswahl-Transistors 806 mit einer Kalibrier-Konstantstromquelle 810 gekoppelt ist. Ist das Umschalt-Element 807 dagegen in der Schalterstellung "a" (nicht gezeigt in der Figur), so ist der zweite Source-/Drain-Anschluss 806b des Auswahl-Transistors 806 mit einer Erfass-Konstantspannungsquelle 811 gekoppelt. Mittels einer Strom-Erfass-Einheit 812, d.h. beispielsweise einem Amperemeter, kann der Wert des auf der Zeilenleitung 809 fließenden elektrischen Stroms erfasst werden.

Ferner ist ein Auswahl-Anschluss 813 mit der ersten Spaltenleitung 808 gekoppelt. Ist an den Auswahl-Anschluss 813 ein elektrisches Signal mit einem logischen Wert "1" angelegt, so sind alle Auswahl-Transistoren 806 der zugehörigen Spalte von Sensor-Einrichtungen 801 elektrisch leitfähig, so dass die zugehörige Spalte von Sensor-Einrichtungen 801c ausgewählt ist. Ferner ist jeweils ein Kalibrier-Anschluss 814 mit einer jeweiligen zweiten Spaltenleitung 815 gekoppelt, wobei für jede Spalte von Sensor-Einrichtungen 801 eine gemeinsame zweite Spaltenleitung 815 und ein gemeinsamer Kalibrier-Anschluss 814 vorgesehen ist. Liegt an dem Kalibrier-Anschluss 814 ein Signal mit einem logischen Wert "1", so sind alle Kalibrier-Transistoren 805 der zugehörigen Spalte von Sensor-Einrichtungen 801 elektrisch leitfähig, so dass ein Kalibrieren ermöglicht ist.

Im Weiteren wird die Funktionalität der Sensor-Anordnung 800 näher beschrieben.

Zunächst wird beschrieben, wie die Sensor-Einrichtungen 801 spaltenweise kalibriert werden, um zwischen unterschiedlichen Sensor-Einrichtungen schwankende Eigenschaften (beispielsweise Schwankungen im Wert der Schwellenspannung der Erfass-Transistoren 804) zu kompensieren. Zum Kalibrieren der n-ten Spalte wird diese als einzige Spalte aktiviert, indem ein Steuersignal mit einem logischen Wert "1" an den Auswahl-Anschluss 813 der n-ten Spalte von Sensor-Einrichtungen 801 angelegt wird. An den Auswahl-Anschlüssen 813 aller anderen Spalten von Sensor-Einrichtungen 801 liegt ein Signal mit einem logischen Wert "0" an. Dadurch sind die Kanal-Bereiche aller Auswahl-Transistoren 806 der n-ten Spalte von Sensor-Einrichtungen 801 elektrisch leitfähig, wohingegen die Auswahl-Transistoren 806 aller anderen Spalten von Sensor-Einrichtungen 801 nicht-leitend sind. Außerhalb einer jeden Zeile von Sensor-Einrichtung 801 befindet sich jeweils eine Auslese- bzw. Kalibrier-Schaltung mit dem Umschalt-Element 807. Zum Durchführen der Kalibrierung wird der Schalter in die Stellung "b" gebracht. Dieses Szenario ist in Fig.8 gezeigt. An den Kalibrier-Anschluss 814 der n-ten Spalte von Sensor-Einrichtungen 801 wird zum Kalibrieren kurzzeitig ein Signal mit einem logischen Wert "1" angelegt, so dass die Kalibrier-Konstantstromquelle 810 über den leitenden Auswahl-Transistor 806 und den leitenden Kalibrier-Transistor 805 mit dem Gate-Anschluss 804c des Erfass-Transistors 804 gekoppelt ist. Dadurch wird ein konstanter Strom $I_{kal}$ in die Sensor-Einrichtung 801 eingeprägt. Ist der Kalibrier-Transistor 805 leitend, so stellt sich am Gate-Anschluss 804c des Erfass-Transistors 804 exakt die elektrische Spannung ein, die erforderlich ist, um den elektrischen Strom $I_{kal}$ durch den Erfass-Transistor 804 abzuleiten. Diese Spannung ist für jede Sensor-Einrichtung 801 der n-ten Spalte von Sensor-Einrichtungen 801 unterschiedlich, da aufgrund statistischer Effekte die elektrischen Parameter des Erfass-Transistors 804 variieren können. Wird die Kopplung des Gate-Anschlusses 804c und des ersten Source-/Drain-Anschlusses 804a des Erfass-Transistors 804 über den Kalibrier-Transistor 805 wieder unterbrochen, indem das Signal an dem Kalibrier-Anschluss 814 auf einen logischen Wert "0" gebracht wird, wird anschaulich die zum elektrischen Stromwert $I_{kal}$ zugehörige elektrische Gate-Spannung an dem Gate-Anschluss 804c des Erfass-Transistors 804 abgespeichert. Dieses Kalibrier-Verfahren wird nach und nach für alle Spalten wiederholt.

Im Weiteren wird eine Messphase der Sensor-Anordnung 800 beschrieben.

Hierzu wird das Umschalt-Element 807 in die Schalterstellung "a" umgelegt (nicht gezeigt in Fig.8). Dadurch wird in alle Schaltkreis-Einrichtungen 801 einer zugehörigen Zeile von Schaltkreis-Einrichtungen 801 die konstante Spannung $V_{drain}$ unter Verwendung der Erfass-Konstantspannungsquelle 811 eingeprägt. Die Sensor-Anordnung 800 wird spaltenweise sequentiell ausgelesen. Eine auszulesende Spalte wird ausgewählt, indem der zugehörige Auswahl-Anschluss 813 auf ein elektrisches Potential mit einem logischen Wert "1" gebracht wird, so dass alle Auswahl-Transistoren 806 der zugehörigen Spalte von Sensor-Anordnungen 801 in einen elektrisch leitfähigen Zustand gebracht werden. Erfolgt an einem Sensor-Element einer Sensor-Einrichtung 801 einer ausgewählten Spalte von Sensor-Einrichtungen 801 kein Sensor-Ereignis, so fließt durch die aktivierte Sensor-Einrichtung 801 derjenige Gleichstrom-Anteil, der auf dem Gate-Anschluss 804c des Erfass-Transistors 804 in der Kalibrierungsphase abgespeichert wurde. Daher sind Parameter-Schwankungen, insbesondere der Erfass-Transistoren 804, kompensiert. Mit anderen Worten ist das Ausgangssignal für ein identisches Sensor-Ereignis gleich und hängt nicht von den schwankenden Parametern der Transistoren ab. Erfolgt infolge eines Sensor-Ereignisses eine Modulation des elektrischen Potentials auf einem Sensor-Element, so resultiert daraus eine Modulation der elektrischen Spannung an dem Gate-Anschluss 804c des zugehörigen Erfass-Transistors 804 und folglich des elektrischen Stroms an dem ersten Source-/Drain-Anschluss 804a des Erfass-Transistors 804. Diese Modulation wird mittels der Strom-Erfass-Einheit 812 erfasst und kann von externen Verstärker-Elementen verstärkt werden. Optional kann das elektrische Sensor-Stromsignal in eine elektrische Spannung umgewandelt werden (vgl. Auswerte-Einheit 700 aus Fig. 7).

Zusammenfassend ist festzuhalten, dass mittels der Sensor-Anordnung 800 die Sensor-Einrichtungen 801 spaltenweise aktiviert bzw. deaktiviert werden können. Ein Sensor-Signal wird verstärkt bzw. in einen elektrischen Strom umgesetzt. Mittels Kalibrierens der Sensor-Einrichtungen 801 werden statistische Schwankungen von Parametern der Sensor-Einrichtungen 801 kompensiert.

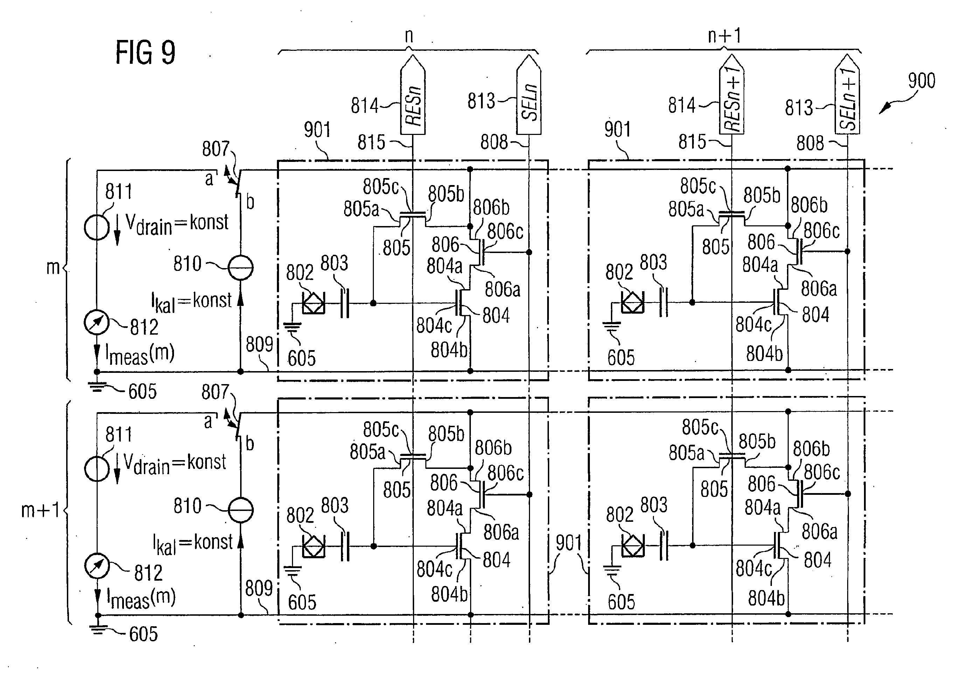

Im Weiteren wird bezugnehmend auf Fig.9 eine Sensor-Anordnung 900 gemäß einem zweiten bevorzugten Ausführungsbeispiel der Erfindung beschrieben. Die in Fig.9 gezeigte Sensor-Anordnung 900 ist eine Modifikation der in Fig.8 gezeigten Sensor-Anordnung 800. Gleiche bzw. ähnliche Komponenten sind daher mit gleichen Bezugsziffern versehen.

Der wesentliche Unterschied zwischen der Sensor-Anordnung 900 und der Sensor-Anordnung 800 ist, dass bei den Sensor-Einrichtungen 901 der Sensor-Anordnung 900 der Kalibrier-Transistor 805 in modifizierter Weise verschaltet ist.

Bei der Sensor-Anordnung 900 ist der zweite Source-/Drain-Anschluss 805b des Kalibrier-Transistors 805 mit dem zweiten Source-/Drain-Anschluss 806b des Auswahl-Transistors 806 und direkt mit der Zeilenleitung 809 gekoppelt. Indem der Kalibrier-Transistor 805 der Sensor-Anordnung 900 direkt mit der Zeilenleitung 809 (anschaulich Ausleseleitung) gekoppelt ist, können geringfügige Schwankungen, welche über den Auswahl-Transistor 806 zustande kommen können, kompensiert werden. Allerdings kann die parasitäre Kapazität am Ausgangsknoten einer Sensor-Anordnung 901 gemäß der Sensor-Anordnung 900 etwas höher sein, da in diesem Fall zwei Transistoren mit ihren Source-/Drain-Anschlüssen permanent am gemeinsamen Ausgangsknoten einer Sensor-Anordnung 901 einer Zeile angeschlossen sind.

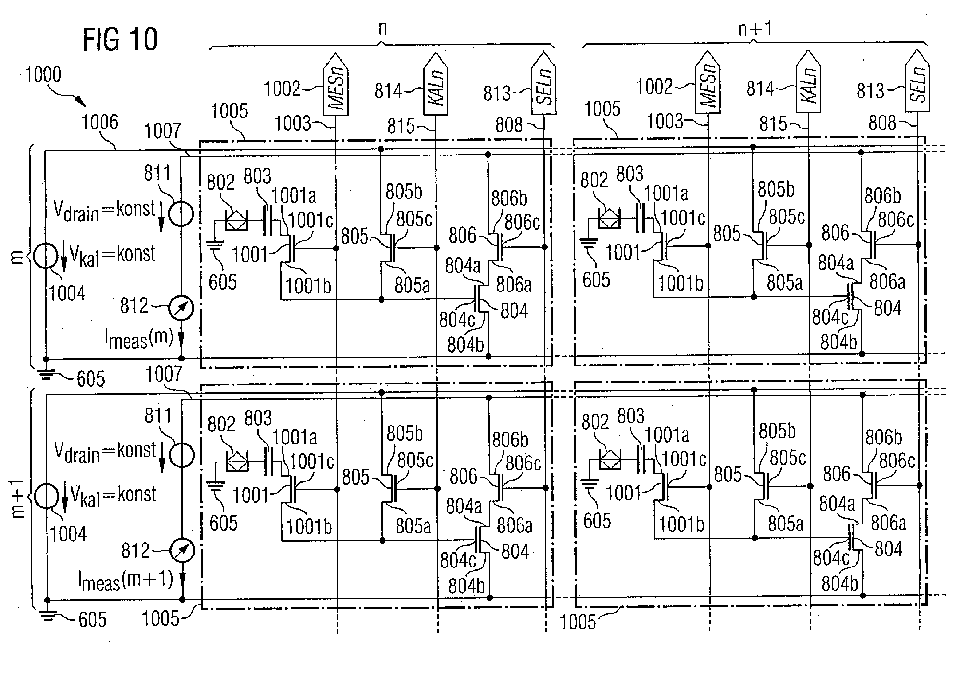

Im Weiteren wird bezugnehmend auf **Fig.10** eine Sensor-Anordnung 1000 gemäß einem dritten bevorzugten Ausführungsbeispiel der Erfindung beschrieben. Diejenigen Komponenten der Sensor-Anordnung 1000, die auch in der Sensor-Anordnung 800 bzw. 900 vorkommen, sind mit gleichen Bezugsziffern versehen.

Die Sensor-Anordnung 1000 ist eine matrixförmige Anordnung einer Vielzahl von Sensor-Einrichtungen 1005.

Wiederum ist ein Sensor-Element einer Sensor-Einrichtung 1005 als Spannungsquelle 802 symbolisiert, die gemäß Fig.10 zwischen einem Anschluss auf elektrischem Massepotential und dem Kondensator 803 geschaltet sind. Der Kondensator 803 stellt eine dielektrische Schicht auf dem Erfass-Transistor 804 dar, mittels dem das Sensor-Element 802 von dem Erfass-Transistor 804 entkoppelt ist. Der Kondensator 803 ist mit dem ersten Source-/Drain-Anschluss 1001a eines Schalt-Transistors 1001 gekoppelt. Der zweite Source-/Drain-Anschluss 1001b des Schalt-Transistors 1001 ist mit dem ersten Source-/Drain-Anschluss 805a des Kalibrier-Transistors 805 und mit dem Gate-Anschluss 804c des Erfass-Transistors 804 gekoppelt. Ferner ist der Gate-Anschluss 1001c des Schalt-Transistors 1001 über eine dritte Spaltenleitung 1003 mit einem Schalt-Anschluss 1002 gekoppelt. Für jede Spalte von Sensor-Einrichtungen 1005 ist eine separate dritte Spaltenleitung 1003 vorgesehen. Der erste Source-/Drain-Anschluss 804a des Erfass-Transistors 804 ist mit dem ersten Source-/Drain-Anschluss 806a des Auswahl-Transistors 806 gekoppelt, dessen Gate-Anschluss 806b über die erste Spaltenleitung 808 mit dem Auswahl-Anschluss 813 gekoppelt ist. Der Gate-Anschluss 805c des Kalibrier-Transistors 805 ist über die zweite Spaltenleitung 815 mit dem Kalibrier-Anschluss 814 gekoppelt. Der zweite Source-/Drain-Anschluss 805b des Kalibrier-Transistors 805 ist mit einer ersten Zeilenleitung 1006 gekoppelt, die für jede Zeile von Sensor-Einrichtungen 1005 gemeinsam vorgesehen ist. Die erste Zeilenleitung 1006 ist mit einer Kalibrier-Konstantspannungsquelle 1004 gekoppelt. Der zweite Source-/Drain-Anschluss 806b des Auswahl-Transistors 806 ist über eine zweite Zeilenleitung 1007 mit der Erfass-Konstantspannungsquelle 811 gekoppelt, welche wiederum mit der Strom-Erfass-Einheit 812 gekoppelt ist. Die Strom-Erfass-Einheit 812 ist mit der Kalibrier-Konstantspannungsquelle 1004 gekoppelt.

Im Weiteren wird die Funktionalität der Sensor-Anordnung 1000 näher beschrieben.

Bei der Sensor-Anordnung 1000 können die Sensor-Einrichtungen 1005 spaltenweise aktiviert bzw. deaktiviert werden. Das Sensor-Signal oder ein Referenz-Signal können verstärkt bzw. in einen elektrischen Strom umgesetzt werden. Ferner eignet sich die Sensor-Einrichtung 1000 für ein Correlated-Double-Sampling zum Eliminieren von Parameterschwankungen.

Es ist anzumerken, dass die n-MOS-Transistoren 1001, 805, 806, die mit einer der Spaltenleitungen 1003, 815 bzw. 808 gekoppelt sind, mittels Anlegens eines elektrischen Signals mit einem logischen Wert "1" an den zugehörigen Anschluss 1002, 814 bzw. 813 in einen elektrisch leitfähigen Zustand gebracht werden können und damit einen vernachlässigbar kleinen ohmschen Widerstand darstellen. Ist hingegen das elektrische Signal an einen zugehörigen Anschluss 1002, 814 bzw. 813 auf einem logischen Wert "0", so ist der angesteuerte Transistor nicht-leitend, wobei die Leckströme der Feldeffekttransistoren vernachlässigt werden können.

Die Sensor-Anordnung 1000 basiert auf dem Correlated-Double-Sampling-Prinzip (CDS-Verfahren), wodurch Parameter-Schwankungen und niederfrequentes Rauschen unterdrückt werden. Gemäß dem CDS-Verfahren wird üblicherweise an einen Eingang eines Verstärkers zunächst ein Referenz-Signal angelegt. Am Ausgang des Verstärkers wird dann das verstärkte Referenz-Signal plus einem Offset-Signal des Verstärkers abgespeichert. In einer nächsten Phase wird das Sensor-Signal an den Verstärker angelegt. Am Ausgang des Verstärkers liegt dann das verstärkte Mess-Signal inklusive dem Offset-Signal an. Mittels einer Differenzbildung der beiden Werte ist es ermöglicht, das Offset-Signal des Verstärkers zu eliminieren.

Die Sensor-Einrichtungen 1005 der Sensor-Anordnung 1000 werden spaltenweise sequentiell ausgelesen. Es wird jeweils eine Spalte (z.B. die n-te Spalte) von Sensor-Einrichtungen 1005 aktiviert, indem der Auswahl-Anschluss 813, die erste Spaltenleitung 808 und folglich die Gate-Anschlüsse 806c der Auswahl-Transistoren 806 der zugehörigen Spalte auf einen logischen Wert "1" gebracht werden. Die Pixel einer Spalte werden gemäß dem Ausführungsbeispiel in zwei Phasen ausgelesen.

In einer ersten Phase wird die Referenz-Spannung $V_{kal}$ der Kalibrier-Konstantspannungsquelle 1004 in einer zugehörigen Sensor-Anordnung 1005 in einen elektrischen Strom umgesetzt und dessen Wert erfasst. Dazu wird an den Kalibrier-Anschluss 814 ein elektrisches Signal mit einem logischen Wert "1" angelegt, so dass die damit über die zweite Spaltenleitung 815 gekoppelten Kalibrier-Transistoren 805 in einen elektrisch-leitfähigen Zustand gebracht werden. Dagegen ist der Schalt-Anschluss 1002 in dieser Phase auf einem logischen Wert "0", so dass der Schalt-Transistor 1001 elektrisch nicht-leitend ist. An dem Gate-Anschluss 804c des Erfass-Transistors 804 liegt die Referenz-Spannung $V_{kal}$ an, was einen zugehörigen elektrischen Strom durch den ersten Source-/Drain-Anschluss 804a des Erfass-Transistors 804 zur Folge hat. Der Wert dieses elektrischen Strom kann aufgrund statistischer Schwankungen von Transistor-Parametern für unterschiedliche Sensor-Einrichtungen 1005 der Sensor-Anordnung 1000 unterschiedlich sein. Der Wert dieses elektrischen Stroms wird in der Auslese-Schaltung der jeweiligen Zeile als elektrischer Referenzstrom $I_{meas}(m)$ erfasst und abgespeichert.

In einer zweiten Phase wird das eigentliche Sensor-Signal erfasst. In einem möglichst kurzen zeitlichen Abstand zu der ersten Phase wird das elektrische Signal an dem Kalibrier-Anschluss 814 auf einen logischen Wert "0" gebracht, wodurch die Kalibrier-Transistoren 805 sperren. Dagegen wird ein elektrisches Signal mit einem logischen Wert "1" an den Schalt-Anschluss 1002 angelegt, so dass die SchaltTransistoren 1001 in einen elektrisch leitfähigen Zustand gebracht werden. Dadurch wird eine Änderung des elektrischen Potentials am Sensor-Element 802 auf dem Gate-Anschluss 804c des Erfass-Transistors 804 abgebildet, was zu einer Modulation des elektrischen Stroms durch den ersten Source-/ Drain-Anschluss 804a des Erfass-Transistors 804 führt. Der Wert dieses elektrischen Stroms wird erfasst, anschließend wird die Differenz zwischen den erfassten Strom-Werten aus der ersten bzw. der zweiten Phase gebildet. Dadurch sind Parameter-Schwankungen zwischen den unterschiedlichen Sensor-Einrichtungen unterdrückt und das erhaltene Ausgangssignal hängt ausschließlich vom Sensor-Ereignis ab.

In diesem Dokument ist folgende Veröffentlichung zitiert:

[1] Stevanovic, N, Hillebrand, M, Hosticka, BJ, Teuner, A (2000) "A CMOS Image Sensor for High-Speed Imaging", IEEE International Solid-State Circuits Conference 2000:104-105

[2] WO 88 08972

[3] US 2001/044177

[4] FR 2 770 826 A

[5] US 4 322 680 A

[6] US 4 514 263 A

[7] EP 0 241 991 A

Bezugszeichenliste

100 Sensor-Anordnung
101 erste Sensor-Einrichtung
102 zweite Sensor-Einrichtung
103 dritte Sensor-Einrichtung
104 vierte Sensor-Einrichtung
105 Multiplexer
106 Auslese-Schaltung
200 Sensor-Anordnung
201 ohmscher Widerstand

202 Kapazität
300 Diagramm
301 Abszisse
302 Ordinate
303 Signalverlauf-Kurve
400 Ausgangstreiber-Schaltung
401 MOS-Transistor
402 Spannungsquelle
403 Konstantstromquelle
404 ohmscher Widerstand
405 Kapazität
406 Versorgungsspannung
407 Massepotential
410 Ersatzschaltbild
411 gesteuerte Stromquelle
412 Innenwiderstand
500 Ausgangstreiber-Schaltung
510 Ersatzschaltbild
511 gesteuerte Stromquelle
512 Innenwiderstand
600 Sensor-Einrichtung
601 Feldeffekttransistor
601a erster Source-/Drain-Anschluss
601b zweiter Source-/Drain-Anschluss
601c Gate-Anschluss
602 Spannungsquelle
603 Amperemeter
604 Versorgungsspannung
605 Massepotential
606 Kondensator
700 Auswerte-Einheit
701 Widerstand
702 Eingang
703 Operationsverstärker
703a invertierender Eingang
703b nicht-invertierender Eingang
703c Ausgang
704 Konstantspannungsquelle
800 Sensor-Anordnung
801 Sensor-Einrichtung
802 Spannungsquelle
803 Kondensator
804 Erfass-Transistor
804a erster Source-/Drain-Anschluss
804b zweiter Source-/Drain-Anschluss
804c Gate-Anschluss
805 Kalibrier-Transistor
805a erster Source-/Drain-Anschluss
805b zweiter Source-/Drain-Anschluss
805c Gate-Anschluss
806 Auswahl-Transistor
806a erster Source-/Drain-Anschluss
806b zweiter Source-/Drain-Anschluss
806c Gate-Anschluss
807 Umschalt-Element
808 erste Spaltenleitung
809 Zeilenleitung
810 Kalibrier-Konstantstromquelle

811 Erfass-Konstantspannungsquelle
812 Strom-Erfass-Einheit
813 Auswahl-Anschluss
814 Kalibrier-Anschluss
815 zweite Spaltenleitung
900 Sensor-Anordnung
901 Sensor-Einrichtung
1000 Sensor-Anordnung
1001 Schalt-Transistor
1001a erster Source-/Drain-Anschluss
1001b zweiter Source-/Drain-Anschluss
1001c Gate-Anschluss
1002,Schalt-Anschluss
1003 dritte Spaltenleitung
1004 Kalibrier-Konstantspannungsquelle
1005 Sensor-Einrichtung
1006 erste Zeilenleitung
1007 zweite Zeilenleitung

**Patentansprüche**

1. Sensor-Anordnung (800, 900, 1000),
mit einer Mehrzahl von auf und/oder in einem Substrat ausgebildeten Sensor-Einrichtungen (801), wobei jede der Sensor-Einrichtungen aufweist

- einen elektrischen Signal-Umsetzer;
- ein mit dem Signal-Umsetzer gekoppeltes Sensor-Element (802), mit dem die elektrische Leitfähigkeit des Signal-Umsetzers in Folge eines Sensor-Ereignisses auf dem Sensor-Element charakteristisch beeinflussbar ist;
- eine Einrichtung (811) zum Konstanthalten einer an dem Signal-Umsetzer anliegenden elektrischen Spannung;
- eine Einrichtung (812) zum Erfassen des Werts des durch den Signal-Umsetzer fließenden elektrischen Stroms als Sensor-Signal;
- wobei der elektrische Signal-Umsetzer ein Feldeffekttransistor (804) ist, dessen Gate-Anschluss (804c) mit dem Sensor-Element gekoppelt ist, wobei die Einrichtung (811) zum Konstanthalten einer elektrischen Spannung derart eingerichtet ist, dass sie die elektrische Spannung zwischen den Source-/Drain-Anschlüssen des Feldeffekttransistors (804) konstant hält; und
- mit einer Kalibrier-Einrichtung (805, 810) zum Kalibrieren einer jeweiligen Sensor-Einrichtung (801), die derart eingerichtet ist, dass mit ihr der Gate-Bereich (804c) des Feldeffekttransistors (804) auf ein derartiges elektrisches Kalibrier-Potential bringbar ist, dass der elektrische Strom von Parameterschwankungen des Feldeffekttransistors (804) unabhängig ist.

2. Sensor-Anordnung nach Anspruch 1,
mit einer Auswerte-Einheit (700), welcher der Wert des elektrischen Stroms als Sensor-Signal bereitgestellt ist.

3. Sensor-Anordnung nach Anspruch 2,
bei der die Auswerte-Einheit (700) derart eingerichtet ist, dass sie aus dem Wert des elektrischen Stroms eine für diesen Wert charakteristische elektrische Spannung bildet oder den Wert des elektrischen Stroms auf einen diesen charakterisierenden digital codierten Wert abbildet.

4. Sensor-Anordnung nach Anspruch 3,
bei der die Auswerte-Einheit einen Operationsverstärker (703) aufweist

- mit einem ersten Eingang (703a), an den das Sensor-Signal anlegbar ist;
- mit einem zweiten Eingang (703b), an den ein elektrisches Referenz-Potential anlegbar ist;
- mit einem Ausgang (703c), an dem die charakteristische elektrische Spannung bereitgestellt ist;
- wobei der erste Eingang (703a) und der Ausgang (703c) mittels eines ohmschen Widerstands (Z 701) miteinander gekoppelt sind.

13

**5.** Sensor-Anordnung nach einem der Ansprüche 1 bis 4, ausgestaltet als Biosensor-Anordnung.

**6.** Sensor-Anordnung nach einem der Ansprüche 1 bis 5,
bei der die Kalibrier-Einrichtung derart eingerichtet ist, dass zum Kalibrieren an den Gate-Anschluss und an einen Source-/Drain-Anschluss des Feldeffekttransistors ein elektrischer Kalibrier-Strom anlegbar ist.

**7.** Sensor-Anordnung nach einem der Ansprüche 2 bis 6, bei der die Auswerte-Einheit eine Correlated-Double-Sampling-Einrichtung aufweist, die derart eingerichtet ist, dass sie bei einem Sensor-Ereignis einen von Parameterschwankungen des Feldeffekttransistors unabhängigen Wert des elektrischen Stroms bildet.

**8.** Sensor-Anordnung nach Anspruch 7,
bei der die Correlated-Double-Sampling-Einrichtung derart eingerichtet ist, dass mit ihr

- in einer Kalibrierphase der Gate-Bereich des Feldeffekttransistors auf ein elektrisches Kalibrier-Potential gebracht wird und der zugehörige Wert des elektrischen Stroms als Kalibrier-Signal erfasst und gespeichert wird;
- in einer Erfassungsphase der Wert des elektrischen Stroms in Folge eines Sensor-Ereignisses als Sensor-Signal erfasst wird;
- in einer Auswertephase Sensor-Signal und Kalibrier-Signal gemeinsam ausgewertet werden.

**9.** Sensor-Anordnung nach einem der Ansprüche 1 bis 8,
bei der die Sensor-Einrichtungen im Wesentlichen matrixförmig auf und/oder in dem Substrat angeordnet sind und mittels Zeilen- und Spalten-Leitungen derart verschaltet sind, dass die Sensor-Einrichtungen einzeln, zeilenweise bzw. spaltenweise ansteuerbar sind.

**10.** Sensor-Anordnung nach Anspruch 9,
bei der mindestens eine Auswerte-Einheit, mindestens eine Kalibrier-Einrichtung und/oder mindestens eine Correlated-Double-Sampling-Einrichtung für zumindest einen Teil der Sensor-Einrichtungen einer Zeilen-Leitung bzw. einer SpaltenLeitung gemeinsam vorgesehen ist/sind.

**11.** Verfahren zum Betreiben einer Sensor-Anordnung (800, 900, 1000),

- mit einer Sensor-Anordnung mit einer Mehrzahl von auf und/oder in einem Substrat ausgebildeten Sensor-Einrichtungen (801, 901, 1001)
- wobei jede der Sensor-Einrichtungen aufweist

-- einen elektrischen Signal-Umsetzer;
-- ein mit dem Signal-Umsetzer gekoppeltes Sensor-Element (802), mit dem die elektrische Leitfähigkeit des Signal-Umsetzers in Folge eines Sensor-Ereignisses auf dem Sensor-Element charakteristisch beeinflussbar ist;
-- eine Einrichtung (811) zum Konstanthalten einer an dem Signal-Umsetzer anliegenden elektrischen Spannung;
-- eine Einrichtung (812) zum Erfassen des Werts des durch den Signal-Umsetzer fließenden elektrischen Stroms als Sensor-Signal;
-- wobei der elektrische Signal-Umsetzer ein Feldeffekttransistor (804) ist, dessen Gate-Anschluss mit dem Sensor-Element (802, 803) gekoppelt ist, wobei die Einrichtung (811) zum Konstanthalten einer elektrischen Spannung derart eingerichtet ist, dass sie die elektrische Spannung zwischen den Source-/Drain-Anschlüssen des Feldeffekttransistors (804) konstant hält;

- wobei gemäß dem Verfahren

-- die elektrische Leitfähigkeit des Signal-Umsetzers infolge eines Sensor-Ereignisses auf dem Sensor-Element (802) charakteristisch beeinflusst wird;
die elektrische Spannung an dem Signal-Umsetzer konstant gehalten wird;
der durch den Signal-Umsetzer fließende elektrische Strom als Sensor-Signal erfasst wird; und
-- bei dem zumindest ein Teil der Sensor-Einrichtungen kalibriert wird, indem der Gate-Bereich (804c) des jeweiligen Feldeffekttransistors (804) auf ein derartiges elektrisches Kalibrier-Potential gebracht wird, dass der Wert des elektrischen Stroms bei einem Sensor-Ereignis von Parameterschwankungen des Feldeffekttransistors (804) unabhängig ist.

**12.** Verfahren nach Anspruch 11,
bei dem unter Verwendung des Correlated-Double-Sampling-Verfahrens bei einem Sensor-Ereignis ein von Parameterschwankungen des Feldeffekttransistors unabhängiger Wert des elektrischen Stroms gebildet wird.

**Claims**

**1.** A sensor arrangement (800, 900, 1000) with a plurality of sensor devices (801) formed on and/or in a substrate, each of the sensor devices comprising

- an electrical signal converter;
- a sensor element (802) coupled to the signal converter, which sensor element can be used to characteristically influence the electrical conductivity of the signal converter on account of a sensor event on the sensor element;
- a device (811) for keeping constant an electrical voltage present at the signal converter;
- a device (812) for detecting the value of the electric current flowing through the signal converter as a sensor signal;
- the electrical signal converter being a field-effect transistor (804) whose gate terminal (804c) is coupled to the sensor element, the device (811) for keeping constant an electrical voltage being set up in such a way that it keeps constant the electrical voltage between the source/drain terminals of the field-effect transistor (804); and
- with a calibration device (805, 810) for calibrating a respective sensor device (801), which is set up in such a way that it can be used to bring the gate region (804c) of the field-effect transistor (804) to an electrical calibration potential such that the electric current is independent of parameter fluctuations of the field-effect transistor (804).

**2.** The sensor arrangement as claimed in claim 1, comprising an evaluation unit (700), which is provided with the value of the electric current as a sensor signal.

**3.** The sensor arrangement as claimed in claim 2,
in which the evaluation unit (700) is set up in such a way that it forms, from the value of the electric current, an electrical voltage characteristic of this value or maps the value of the electric current onto a digitally coded value that characterizes the electric current.

**4.** The sensor arrangement as claimed in claim 3,
in which the evaluation unit comprises an operational amplifier (703) comprising

- a first input (703a) to which the sensor signal can be applied;
- a second input (703b) to which an electrical reference potential can be applied;
- an output (703c) at which the characteristic electrical voltage is provided;
- the first input (703a) and the output (703c) being coupled to one another by means of an ohmic resistor (Z 701).

**5.** The sensor arrangement as claimed in one of the claims 1 to 4, configured as a biosensor arrangement.

**6.** The sensor arrangement as claimed in one of the claims 1 to 5,
in which the calibration device is set up in such a way that for calibration an electric calibration current can be applied to the gate terminal and to a source/drain terminal of the field-effect transistor.

**7.** The sensor arrangement as claimed in one of the claims 2 to 6,
in which the evaluation unit comprises a correlated double sampling device which is set up in such a way that it forms, in the case of a sensor event, a value of the electric current that is independent of parameter fluctuations of the field-effect transistor.

**8.** The sensor arrangement as claimed in claim 7,
in which the correlated double sampling device is set up in such a way that, by means of this device,

- in a calibration phase, the gate region of the field-effect transistor is brought to an electrical calibration potential and the associated value of the electric current is detected as a calibration signal and is stored;
- in a detection phase, the value of the electric current on account of a sensor event is detected as a sensor signal;
- in an evaluation phase, the sensor signal and the calibration signal are evaluated jointly.

**9.** The sensor arrangement as claimed in one of the claims 1 to 8,
in which the sensor devices are arranged essentially in matrix form on and/or in the substrate and are connected by means of row lines and column lines in such a way that the sensor devices can be driven individually, row by row or column by column.

**10.** The sensor arrangement as claimed in claim 9,
in which at least one evaluation unit, at least one calibration device and/or at least one correlated double sampling device is/are provided for at least a portion of the sensor devices of a row line or a column line jointly.

**11.** A method for operating a sensor arrangement (800, 900, 1000)

- with a sensor arrangement comprising a plurality of sensor devices (801, 901, 1001) formed on and/or in a substrate,
- each of the sensor devices comprising

-- an electrical signal converter;
-- a sensor element (802) coupled to the signal converter, which sensor element can be used to characteristically influence the electrical conductivity of the signal converter on account of a sensor event on the sensor element;
-- a device (811) for keeping constant an electrical voltage present at the signal converter;
-- a device (812) for detecting the value of the electric current flowing through the signal converter as a sensor signal;
-- the electrical signal converter being a field effect transistor (804) whose gate terminal is coupled to the sensor element (802, 803), the device (811) for keeping constant an electrical voltage being set up in such a way that it keeps constant the electrical voltage between the source/drain terminals of the field-effect transistor (804);

- wherein, in accordance with the method,

-- the electrical conductivity of the signal converter is characteristically influenced on account of a sensor event on the sensor element (802);
-- the electrical voltage at the signal converter is kept constant;
-- the electric current flowing through the signal converter is detected as a sensor signal; and
-- in which at least a portion of the sensor devices is calibrated by the gate region (804c) of the respective field-effect transistor (804) being brought to an electrical calibration potential such that the value of the electric current in the case of a sensor event is independent of parameter fluctuations of the field-effect transistor (804).

**12.** The method as claimed in claim 11,
in which a value of the electric current that is independent of parameter fluctuations of the field-effect transistor is formed using the correlated double sampling method in the case of a sensor event.

**Revendications**

**1.** Système ( 800, 900, 1000 ) détecteur
comprenant une pluralité de dispositifs (801) de détecteurs formés sur et/ou dans un substrat, chacun des dispositifs détecteurs ayant

- un convertisseur électrique de signal ;
- un élément (802) de détecteur, qui est couplé au convertisseur de signal et par lequel la conductivité électrique du convertisseur de signal peut être influencée de manière caractéristique à la suite d'un événement de détecteur sur l'élément de détecteur ;
- un dispositif ( 811 ) pour maintenir constante une tension électrique s'appliquant au convertisseur de signal ;
- un dispositif ( 812 ) pour relever la valeur du courant électrique passant dans le convertisseur de signal en tant que signal de détecteur ;
- dans lequel le convertisseur électrique de signal est un transistor ( 804 ) à effet de champ dont la borne (804c) de grille est couplée à l'élément de détecteur, le dispositif ( 811 ) pour maintenir constante une tension électrique étant tel qu'il maintient constante la tension électrique entre les bornes de source/drain du transistor ( 804 ) à effet de champ ; et

- comprenant un dispositif (805, 810) d'étalonnage pour étalonner un dispositif ( 801 ) de détecteur respectif qui est tel que par lui, la zone ( 804c ) de grille du transistor ( 804 ) à effet de champ peut être mise à un potentiel d'étalonnage électrique tel que le courant électrique est indépendant de fluctuations de paramètres du transistor ( 804 ) à effet de champ.

**2.** Système de détecteur suivant la revendication 1,
comprenant une unité ( 700 ) d'exploitation qui met à disposition la valeur du courant électrique en tant que signal de détecteur.

**3.** Système de détecteur suivant la revendication 2,
dans lequel l'unité ( 700 ) d'exploitation est telle qu'elle forme, à partir de la valeur du courant électrique, une tension électrique caractéristique de cette valeur ou qu'elle reproduit la valeur du courant électrique sur une valeur codée numérique la caractérisant.

**4.** Système de détecteur suivant la revendication 3,
dans lequel l'unité d'exploitation a un amplificateur ( 703 ) opérationnel

- ayant une première entrée ( 703a ) à laquelle le signal de détecteur peut être appliqué ;
- ayant une deuxième entrée ( 703b ) à laquelle un potentiel de référence électrique peut être appliqué ;
- ayant une sortie ( 703c ) sur laquelle la tension électrique caractéristique est disponible ;
- dans lequel la première entrée ( 703a ) et la sortie ( 703c ) sont couplées entre elles au moyen d'une résistance ( Z 701 ) ohmique.

**5.** Système de détecteur suivant l'une des revendications 1 à 4, constitué en système de biodétecteur.

**6.** Système de détecteur suivant l'une des revendications 1 à 5, dans lequel le dispositif d'étalonnage est tel qu'il peut être appliqué, pour l'étalonnage à la borne de grille et à une borne de source/drain du transistor à effet de champ, un courant électrique d'étalonnage.

**7.** Système de détecteur suivant l'une des revendications 2 à 6, dans lequel l'unité d'exploitation a un dispositif correlated-double-sampling qui est tel qu'il forme, pour un événement de détecteur, une valeur du courant électrique indépendante des fluctuations de paramètres du transistor à effet de champ.

**8.** Système de détecteur suivant la revendication 7,
dans lequel le dispositif correlated-double-sampling est tel que par lui

- dans une phase d'étalonnage, la zone de grille du transistor à effet de champ est mise à un potentiel électrique d'étalonnage et la valeur associée du courant électrique est relevée en tant que signal d'étalonnage et mémorisée ;
- dans une phase de relevé, la valeur du courant électrique est relevée en tant que signal de détecteur à la suite d'un événement de détecteur ;
- dans une phase d'exploitation, le signal de détecteur et le signal d'étalonnage sont exploités en commun.

**9.** Système de détecteur suivant l'une des revendications 1 à 8,
dans lequel les dispositifs de détecteurs sont disposés sensiblement sous forme de matrices sur et/ou dans le substrat et sont câblés au moyen de lignes de rangées et de colonnes, de façon à ce que les dispositifs de détecteurs puissent être commandés individuellement, rangée par rangée ou colonne par colonne.

**10.** Système de détecteur suivant la revendication 9,
dans lequel il est prévu en commun au moins une unité d'exploitation, au moins un dispositif d'étalonnage et/ou au moins un dispositif correlated-double-sampling pour au moins une partie des dispositifs de détecteurs d'une ligne de rangées ou d'une ligne de colonnes.

**11.** Procédé pour faire fonctionner un système ( 800, 900, 1000 ) de détecteur,

- comprenant un système de détecteur ayant une pluralité de dispositifs ( 801, 901, 1001 ) de détecteurs formés sur un substrat et/ou dans un substrat

- dans lequel chacun des dispositifs de détecteurs comprend

-- un convertisseur électrique de signal ;

-- un élément ( 802 ) de détecteur, qui est couplé au convertisseur de signal et par lequel la conductivité électrique du convertisseur de signal peut être influencée de manière caractéristique à la suite d'un événement de détecteur sur l'élément de détecteur ;

-- un dispositif ( 811 ) pour maintenir constante une tension électrique s'appliquant au convertisseur de signal ;

-- un dispositif ( 812 ) pour relever la valeur du courant électrique passant dans le convertisseur de signal en tant que signal de détecteur ;

-- dans lequel le convertisseur électrique de signal est un transistor ( 804 ) à effet de champ dont la borne de grille est couplée à l'élément ( 802, 803 ) de détecteur, le dispositif ( 811 ) pour maintenir constante une tension électrique étant tel qu'il maintient constante la tension électrique entre les bornes de source/drain du transistor ( 804 ) à effet de champ ;

- dans lequel, suivant le procédé,

-- la conductivité électrique du convertisseur de signal est influencée de manière caractéristique en raison d'un événement de détecteur sur l'élément ( 802 ) de détecteur ;

-- la tension électrique est maintenue constante sur le convertisseur de signal ;

-- le courant électrique passant dans le convertisseur de signal est relevé en tant que signal de détecteur ; et

-- dans lequel au moins une partie des dispositifs de détecteurs est étalonnée en mettant la zone ( 804c ) de grille du transistor ( 804 ) à effet de champ respectif à un potentiel d'étalonnage électrique tel que la valeur du courant électrique pour un événement de détecteur est indépendante des fluctuations de paramètres du transistor ( 804 ) à effet de champ.

12. Procédé suivant la revendication 11,
dans lequel, en utilisant le procédé correlated-double-sampling pour un événement de détecteur, on forme une valeur du courant électrique qui est indépendante des fluctuations de paramètres du transistor à effet de champ.

FIG 1    Stand der Technik

FIG 2    Stand der Technik

FIG 3

## FIG 4A
Stand der Technik

## FIG 4B
Stand der Technik

## FIG 5A
Stand der Technik

## FIG 5B
Stand der Technik

## FIG 6

604

603 — $I_{out}$

600

601a

601c

602

$V_G \sim$

601 601b

606

C

$V_{out}=konst$

605 605 605

## FIG 7

701

Z

700

$\Delta I_{meas}$

703a 703

703b

702

$+$

703c

$\Delta V_{out}=\Delta I_{meas}\cdot Z$

704 — $V_{kcal}=konst$

605 605

FIG 8

EP 1 554 569 B1

FIG 9

EP 1 554 569 B1

FIG 10

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 8808972 A
- US 2001044177 A
- FR 2770826 A
- US 4322680 A
- US 4514263 A
- EP 0241991 A

**In der Beschreibung aufgeführte Nicht-Patentliteratur**

- **STEVANOVIC, N ; HILLEBRAND, M ; HOSTICKA, BJ ; TEUNER, A.** A CMOS Image Sensor for High-Speed Imaging. *IEEE International Solid-State Circuits Conference,* 2000, 104-105